# EUROPEAN PATENT SPECIFICATION

(11) **EP 3 984 983 B1**
(45) Date of publication and mention of the grant of the patent: **26.11.2025**
(21) Application number: 20201565.7
(22) Date of filing: 13.10.2020
(51) Int. Cl.: C07C 1/04, C07C 1/12, C10L 3/08

(54) **METHANATION WITH TURBOCHARGER**
METHANISIERUNG MIT TURBOLADER
MÉTHANATION À L'AIDE D'UN TURBOCOMPRESSEUR

(43) Date of publication of application: 20.04.2022
(73) Proprietor: Technische Universität München, 80333 München (DE)
(72) Inventor: HERRMANN, Stephan, 81925 München (DE); FISCHER, Felix, 80336 München (DE); SPLIETHOFF, Hartmut, 82140 Olching (DE)
(74) Representative: Hoefer & Partner Patentanwälte mbB

(56) References cited:
- WO-A1-2016/146815
- KR-A- 20150 085 370

## Description

### Field of the invention

The present invention relates to an improved methanation process, wherein energy released during the methanation process is used to drive a turbocharger to drive and/or maintain the process. The present invention is further concerned with a system for the production of methane-enriched gas and optionally power from hydrogen and carbon-containing starting materials comprising at least one methanation reactor and at least one turbocharger.

### Background

Methanation is the conversion of carbon monoxide (CO) and/or carbon dioxide (CO₂) to methane (CH₄) through hydrogenation. This process has been discovered in the early 20^{th} century. Exemplary reactions describe the methanation of carbon monoxide and carbon dioxide respectively:

CO + 3 H₂ -> CH₄ + H₂O

4 H₂ + CO₂ -> CH₄ + 2 H₂O

The methanation reactions are classified as exothermic.

Examples of possible starting materials are CO₂ from biogas plants and H₂ from high-temperature electrolysis plants. Examples for methanation processes of the prior art include the Topsøe Recycle Energy-efficient Methanation Process (TREMP^{™}) by Haldor Topsøe. The TREMP^{™} process can operate at high temperature, up to 973 K, due to the MCR-2X methanation catalyst used in this process. This catalyst allows reaction heat recovery in the form of high-pressure superheated steam and low recycle ratio to ensure energy saving. CO methanation takes place in adiabatic fixed bed reactors. The reaction exothermicity results in a high temperature increase. The reaction product, a methane-enriched gas, generated in the process is partly recycled to control the temperature rise in the methanation reactor. Within the last decade, the interest in substitute natural gas has become strong. Efforts have been reinitiated in the technology, and the knowledge gained over the years has been used to refine the tried and tested technology and catalyst. A demonstration plant (Westfield Coal Gasification plant) was built in Scotland, producing 2.46 million Nm³/h of SNG from coal. The methanation unit used in this plant was composed of fixed bed reactors with gas recycling.

A further development of the British Gas Corporation was the HICOM process, in which a methane-rich gas is made directly from purified gasifier product gas by reaction with steam over a catalyst; the temperature rise is controlled by hot-gas recycling and split-stream operation.

In Germany, Linde developed an isothermal fixed bed reactor with indirect heat exchange. The reactor itself is able to produce steam from the heat of the exothermic methanation reaction. A part of the steam is added to the syngas mixture to minimize the risk of carbon deposition, before being fed into the isothermal and adiabatic methanation reactors.

All processes described above use fixed bed reactors with recycling of the cooled product gas and/or adding steam. This is necessary in order to efficiently dissipate the high reaction enthalpy (approx. 20% of the calorific value of the feed gases) and, if necessary, to use it profitably, in particular for steam generation and optionally generation of superheated steam.

In smaller systems, on the other hand, a single-stage, almost isothermal reactor arrangement is often used, often in combination with a thermal power plant. The heat produced during the methanation process is used to heat the working medium in the thermal power plant. Usually, the working medium is water, and a boiling water reactor-setup is used to control the reaction temperature in the methanation reactor. Alternative working media are thermal oil and molten salt. The water is heated to generate steam. The steam is then mostly expanded in a steam turbine to generate electricity.

CO/CO₂ methanation has many practical applications. It is a means of carbon oxide removal from process gases and it is also considered as an alternative to the PReferential OXidation (PROX) of a carbon monoxide in a gas mixture by a catalyst in fuel processors for mobile fuel cell applications. Methanation as a means of producing synthetic natural gas has been considered since the 1970s. More recently, it has been considered as a way to store energy produced from solar or wind power using power-to-gas systems in conjunction with existing natural gas storage. As part of the energy transition, there will likely be an increased need for seasonal energy storage. It is expected that 30 TWh of storage capacity will be required in Germany if 80% of the electricity is generated with renewable energies.

The power-to-gas process, in which energy is stored by using excess power to synthesize gas, is the most promising technology for seasonal energy storage. The main storage media are hydrogen and methane. Methane has the advantage over hydrogen that the existing infrastructure (gas pipes and natural gas tanks) can be used. In 2018, there were 128 research and demonstration plants for power-to-gas processes in Europe, which shows the great interest in this technology.

To provide an economically viable and technically feasible methanation process, the methane produced must meet several requirements. Methane produced in the methanation process needs to have a high purity, because methane with insufficient purity must not be fed into the natural gas network. Furthermore, methane must be pressurized prior to being fed into the natural gas network. Therefore, a satisfactory methanation process must produce pressurized methane of high purity.

However, possible sources of the starting materials for a methanation process are biogas plants providing CO₂ and high-temperature electrolysis plants providing H₂. These plants usually provide their products with low pressure. Therefore, some methanation plants of the prior art operate at low pressure, which leads to methane of lower purity. This methane needs to be purified and pressurized prior to being fed into the natural gas network.

Therefore, processes have been developed to produce pressurized methane by compressing the starting materials. Methanation is often carried out at elevated pressures to shift the equilibrium towards methane according to Le Chatelier's principle. Depending on the application, the goal is usually a maximum residual hydrogen content of 2-5%. For feed gases with an initially low inlet pressure, a motor-driven compressor is mostly used for this. To improve the equilibrium position, a (partial) intermediate condensation of water vapor formed between two methanation stages may also be provided. This shifts the balance to the right side of the equation accordingly.

Pressurizing the starting materials to be able to obtain pressurized pure methane requires energy. In the prior art, motor-driven compressors requiring external energy, such as electrical energy, are usually employed to pressurize the starting material. The costs associated with this part of the process make up to 50 % of the total cost of the system. Therefore, there is a need for a process, which minimizes the costs associated with the process of pressurizing the starting materials. To achieve this goal, the energy needs to be used efficiently to make the process economically and technically viable.

Therefore, there is a need for an improved methanation process and system, which use the energy provided by the exothermic reaction efficiently, minimize the need for the use of external energy, and thereby efficiently provide pressurized and methane-enriched gas that is sufficiently pure to be fed into the natural gas network. KR20150085370 discloses a gasifier for generating synthetic natural gas. WO2016146815 discloses a process for producing methane and power.

### Summary of the invention

The present invention provides an improved methanation process and system, which use the energy provided by the exothermic reaction efficiently, minimize the need for the use of external energy, and thereby efficiently provide pressurized and methane-enriched gas up to pure methane that can be fed into the natural gas network or used in other downstream applications.

The present invention uses a turbocharger driven by the reaction heat generated in a methanation reactor to pressurize the starting material and/or the methane-enriched gas generated in the system, thereby significantly reducing the costs that would be generated when using a motor-driven compressor.

The improved methanation process and system of the present invention use a novel combination of one or more methanation stages and one or more turbochargers, which can be supplemented with heat exchangers. The methanation process can be an adiabatic process in all stages. The methanation process can however also be isothermal in one or more of the stages.

The improved methanation process and system of the present invention can be achieved in several ways.

In one embodiment, the gas to be methanized (feed gas) or the methane-enriched (methanized) gas (product gas) flows completely through both components of the turbocharger.

In another embodiment, the gas to be methanized or the methanized gas flows only through the compressor of the turbocharger.

In one embodiment of the present invention, the methanized gas is not fed back into the methanation reactor, in which it was produced. The methanized gas proceeds downstream through the continuous gas line and is removed completely from the system. In other words, all of the methanized gas produced can be removed at the exit of the continuous gas line. Removing all of the methanized gas from the system means that methane-enriched gas produced in the process is not returned from the exit of the continuous gas line into one or more of the methanation reactors. Avoiding recirculation simplifies the system structure and avoids having to use a relatively expensive high-temperature compressor or an intermediate cooling step as in the TREMP process of the prior art.

In another embodiment, a part of the methane-enriched gas can be recycled and fed back into the methanation reactor to control the reaction temperature.

In another embodiment, a part of the methane-enriched gas can be recycled by means of a simple control valve without the installation of an additional recycle compressor. This is possible because the pressure at the outlet of the system is higher than at the inlet due to the compression of the main gas flow by the turbocharger.

The main advantage of the present invention is that an increased pressure at the outlet of the system is obtained compared to the initial pressure of the starting materials. The pressurized methane-enriched gas can be directly used for downstream applications. It can for example be fed into the natural gas network without the need of further pressurizing the methane-enriched gas. The process and system of the present invention can therefore use starting materials at low pressure, such as atmospheric pressure, and still provide pressurized methane-enriched gas. Therefore, the process and system of the present invention do not require starting materials at a high pressure and can therefore avoid high process costs associated or caused for example by using electrical energy for a compressor to pressurize the starting materials. Furthermore, with the process and system of the present invention, there is no need for further pressurizing methane-enriched gas prior to its use in downstream processes. Costs associated with this pressurizing step are therefore also avoided.

Thus, the present invention provides an improved methanation process and system, which use the energy provided by the exothermic reaction efficiently, minimize the need for the use of external energy, and thereby efficiently provide pressurized and methane-enriched gas up to pure methane that can be fed into the natural gas network.

### Brief description of the drawings

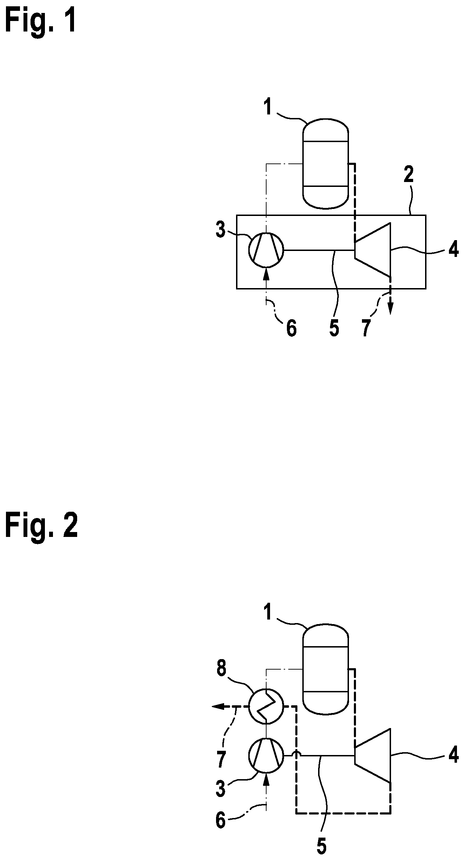

**Figure** 1 shows a schematic for a system for the production of methane-enriched gas from hydrogen and carbon-containing starting materials according to the invention. The system comprises a turbocharger (2) and an integrated adiabatic methanation reactor (1).
**Figure** 2 shows a schematic for a system for the production of methane-enriched gas from hydrogen and carbon-containing starting materials according to the invention. The system comprises a turbocharger, an integrated adiabatic methanation reactor and a heat exchanger (8). The heat exchanger is a recuperator, wherein the produced methane-enriched gas heats the hydrogen and carbon-containing starting materials exiting the compressor (3).

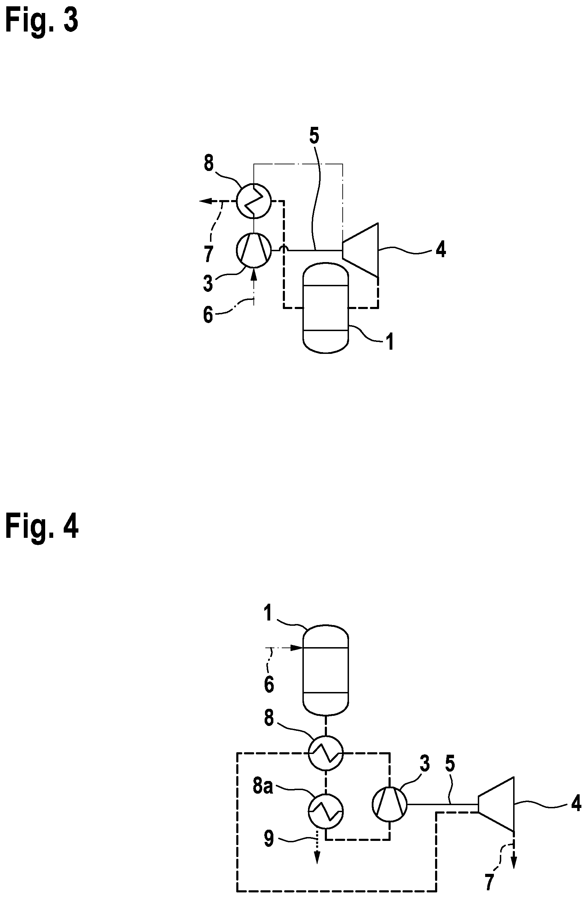

**Figure** 3 shows a schematic for a system for the production of methane-enriched gas from hydrogen and carbon-containing starting materials according to the invention. The system comprises a turbocharger, a methanation reactor (1) arranged downstream of the turbocharger, and a heat exchanger (8). The heat exchanger is a recuperator, wherein the produced methane-enriched gas heats the hydrogen and carbon-containing starting materials exiting the compressor.
**Figure 4** shows a schematic for a system for the production of methane-enriched gas from hydrogen and carbon-containing starting materials according to the invention. The system comprises a turbocharger, and a methanation reactor (1) arranged upstream of the turbocharger. The system further comprises two heat exchangers (8).

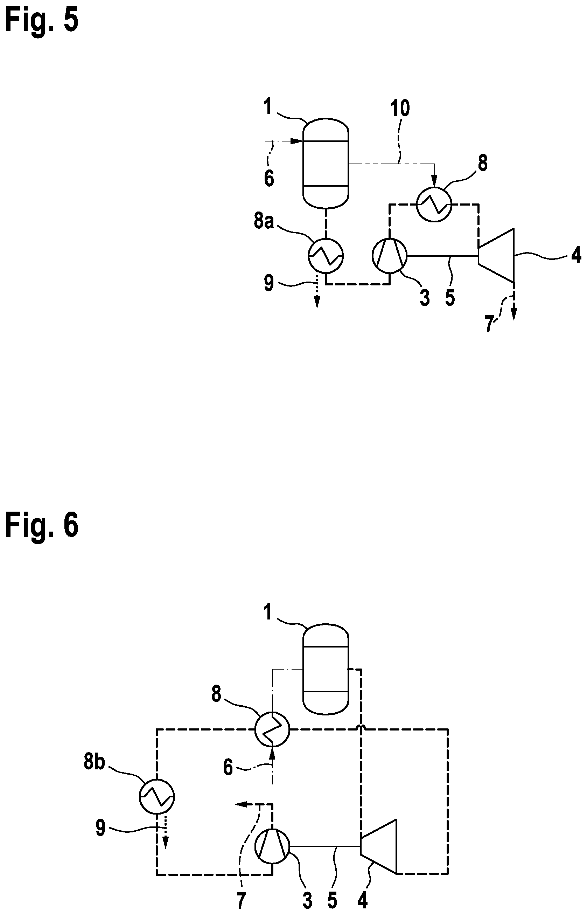

**Figure 5** shows a schematic for a system for the production of methane-enriched gas from hydrogen and carbon-containing starting materials according to the invention. The system is similar to the system shown **Figure 4**. Heat flow (10) from the methanation reaction is used for recuperation of the produced methane-enriched gas exiting the compressor prior to entering the turbine.
**Figure 6** shows a schematic for a system for the production of methane-enriched gas from hydrogen and carbon-containing starting materials according to the invention. The system comprises a turbocharger with an inverted flow of the produced methane-enriched gas, which enters the turbine prior to entering the compressor of the turbocharger.

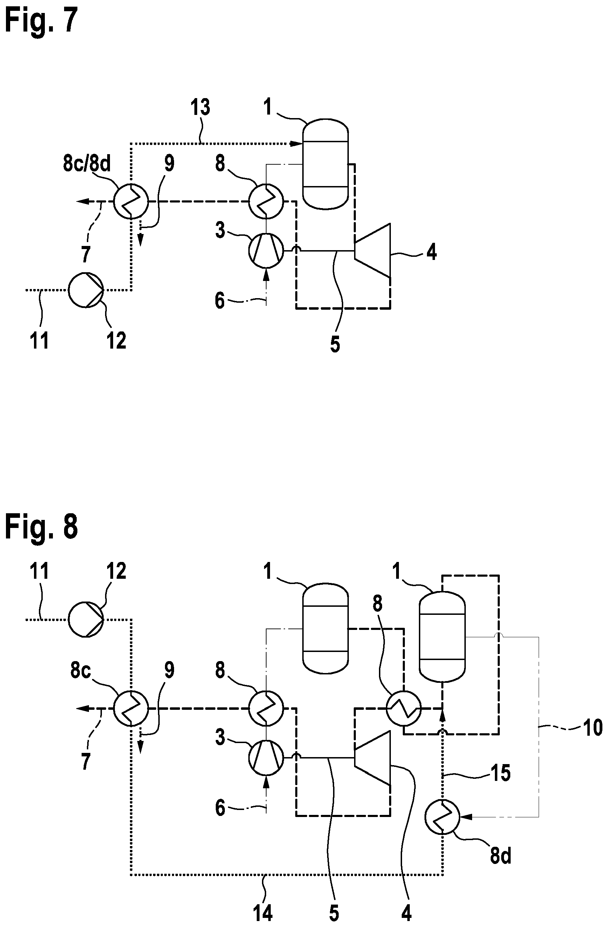

**Figure 7** shows a schematic for a system for the production of methane-enriched gas from hydrogen and carbon-containing starting materials according to the invention similar to the system of **Figure 1**. It further comprises an injection of water (11), which is pressurized by pump (12) and pre-heated and/or partly or fully evaporated in pre-heating element/evaporator (8c/8d) into the methanation reactor for controlling the temperature and increasing the mass flow.
**Figure 8** shows a schematic for a system for the production of methane-enriched gas from hydrogen and carbon-containing starting materials according to the invention similar to the system of **Figure 7**, but comprising two methanation reactors. The first methanation reactor is an adiabatic methanation reactor; the second is an isothermal methanation reactor.

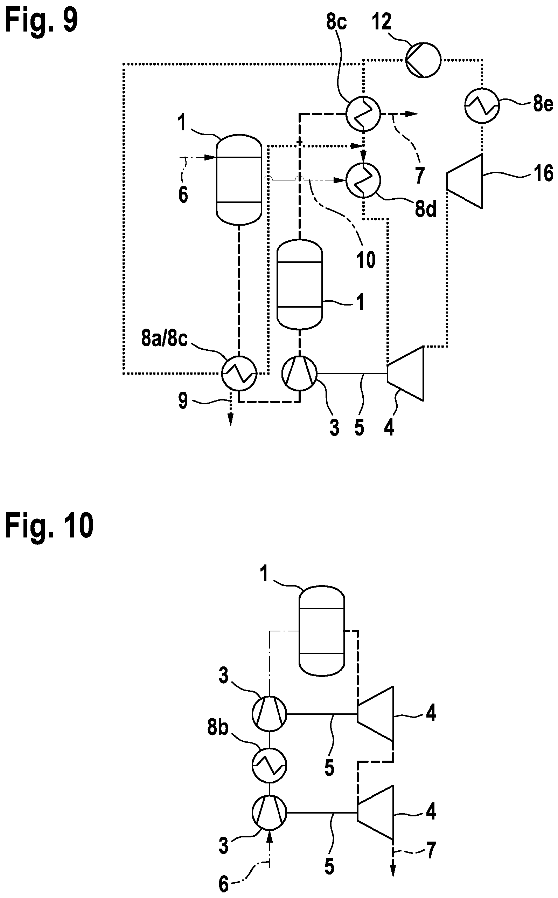

**Figure 9** shows a schematic for a system for the production of methane-enriched gas and power from hydrogen and carbon-containing starting materials according to the invention comprising two methanation reactors. The first methanation reactor is cooled by a boiling water cooler. The methanation process in the first methanation reactor is isothermal. The second methanation reactor is an adiabatic methanation reactor.
**Figure 10** shows a schematic for a system for the production of methane-enriched gas from hydrogen and carbon-containing starting materials according to the invention similar to the system of **Figure 1**, but comprising two turbochargers, wherein an intermediate cooling element (8b) is arranged between the compressors of the respective turbochargers.

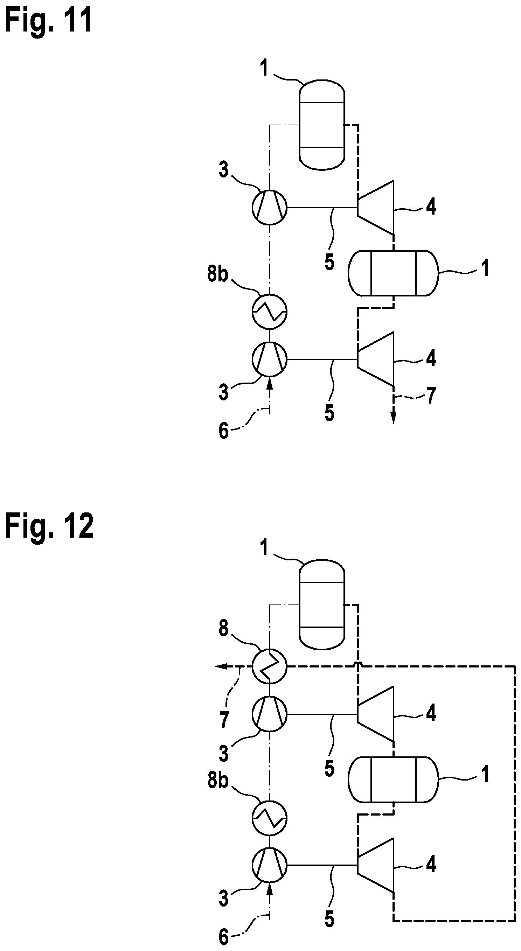

**Figure 11** shows a schematic for a system for the production of methane-enriched gas and power from hydrogen and carbon-containing starting materials according to the invention similar to the system of **Figure 10**, but comprising a second adiabatic methanation reactor arranged between the turbines of the respective turbochargers.
**Figure 12** shows a schematic for a system for the production of methane-enriched gas from hydrogen and carbon-containing starting materials according to the invention similar to the system of **Figure 11**, but comprising an additional recuperator.

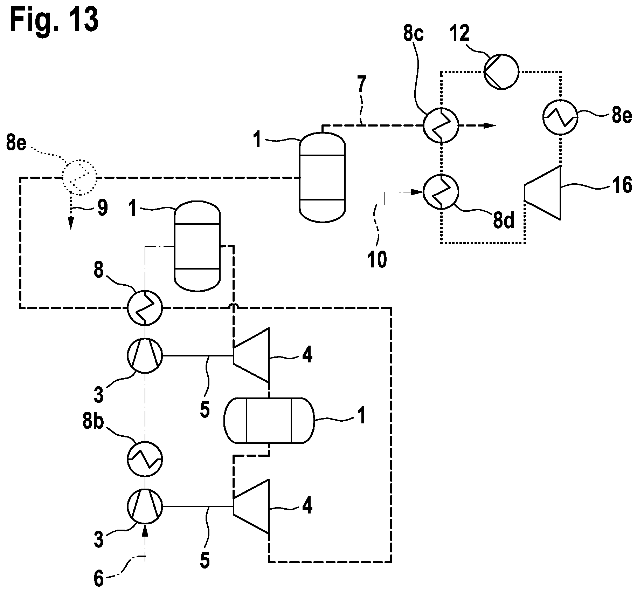

**Figure 13** shows a schematic for a system for the production of methane-enriched gas and power from hydrogen and carbon-containing starting materials according to the invention similar to the system of **Figure 12**, but comprising a third methanation reactor, wherein an isothermal methanation process occurs, and a thermal power plant for production of power.

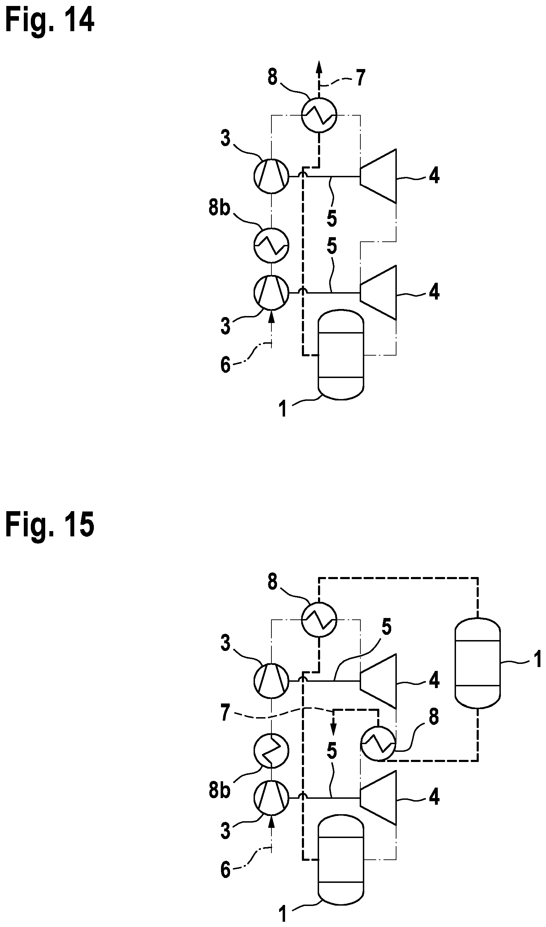

**Figure 14** shows a schematic for a system for the production of methane-enriched gas from hydrogen and carbon-containing starting materials according to the invention comprising two turbochargers, wherein both turbochargers are arranged upstream of an adiabatic methanation reactor and heat is transferred from the product gas to the feed gas with a recuperator before entering the first turbine.
**Figure 15** shows a schematic for a system for the production of methane-enriched gas from hydrogen and carbon-containing starting materials according to the invention similar to the system of **Figure 14**, but comprising an additional adiabatic methanation reactor and an additional recuperator.

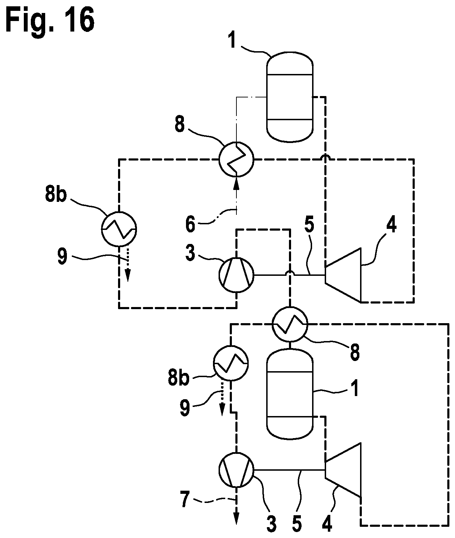

**Figure 16** shows a schematic for a system for the production of methane-enriched gas from hydrogen and carbon-containing starting materials according to the invention comprising a two-stage methanation process with recuperation and turbochargers, which are located downstream of their respective methanation reactors, wherein the methane-enriched gas produced in each methanation reactor enters the turbine prior to entering the compressor (inverted flow).

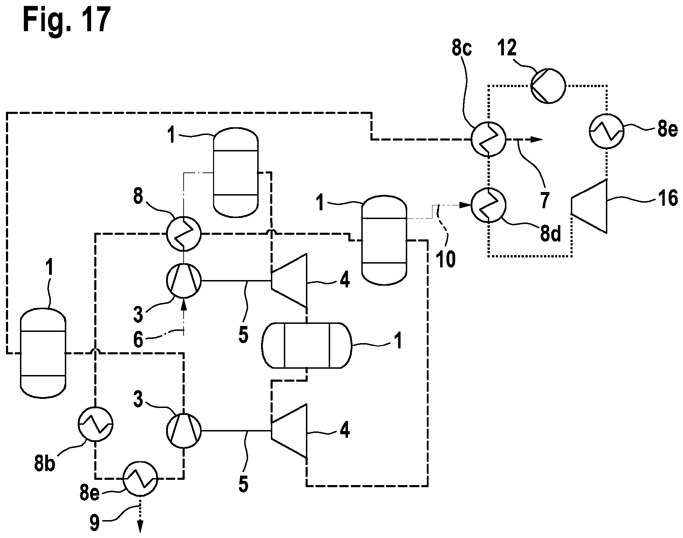

**Figure 17** shows a schematic for a system for the production of methane-enriched gas and power from hydrogen and carbon-containing starting materials according to the invention, which is a combination of a first stage corresponding to the system of **Figure 1**, a second stage with an inverted flow of the methane-enriched gas produced in the second methanation reactor in a downstream turbocharger, a third stage comprising a third methanation reactor with an isothermal methanation process controlled by a steam circuit, and a fourth methanation reactor as polished to obtain a very high methane content in the methane-enriched gas exiting the system.

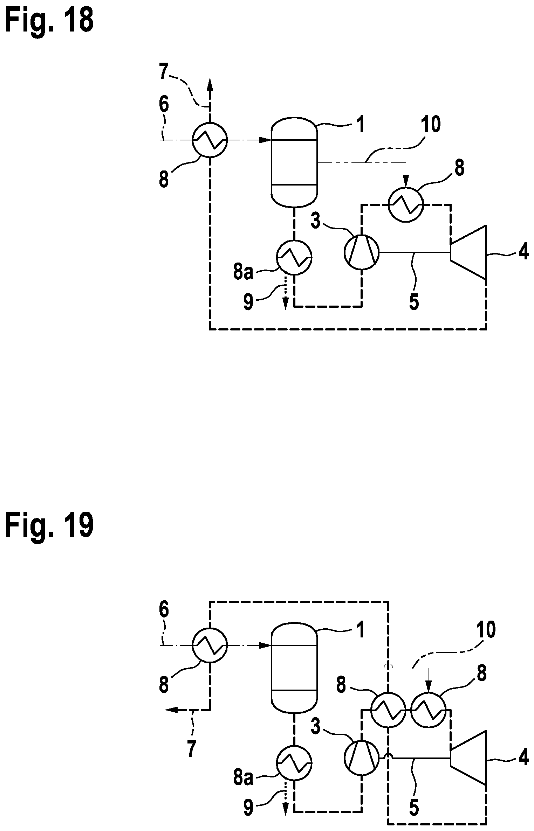

**Figure 18** shows a schematic for a system for the production of methane-enriched gas from hydrogen and carbon-containing starting materials according to the invention, comprising a turbocharger located downstream of the methanation reactor.
**Figure 19** shows a schematic for a system for the production of methane from hydrogen and carbon-containing starting materials according to the invention similar to the system of **Figure 18** comprising an additional recuperator arranged between the compressor and the heat exchanger using the heat flow of the methanation reactor.

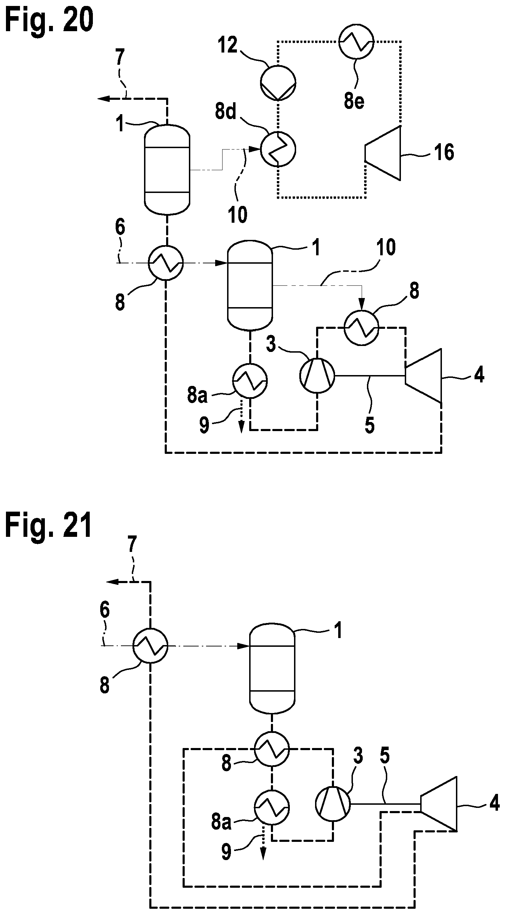

**Figure 20** shows a schematic for a system for the production of methane-enriched gas and power from hydrogen and carbon-containing starting materials according to the invention similar to the system of **Figure 18** comprising an additional methanation stage.
**Figure 21** shows a schematic for a system for the production of methane-enriched gas from hydrogen and carbon-containing starting materials according to the invention similar to the system of **Figure 18**, but comprising an adiabatic methanation reactor and a downstream recuperator.

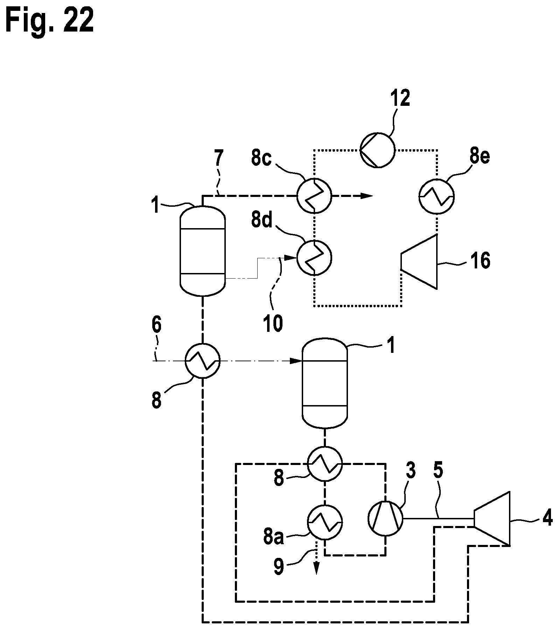

**Figure 22** shows a schematic for a system for the production of methane-enriched gas and power from hydrogen and carbon-containing starting materials according to the invention similar to the system of **Figure 21** comprising an additional isothermal methanation stage and a thermal power plant.

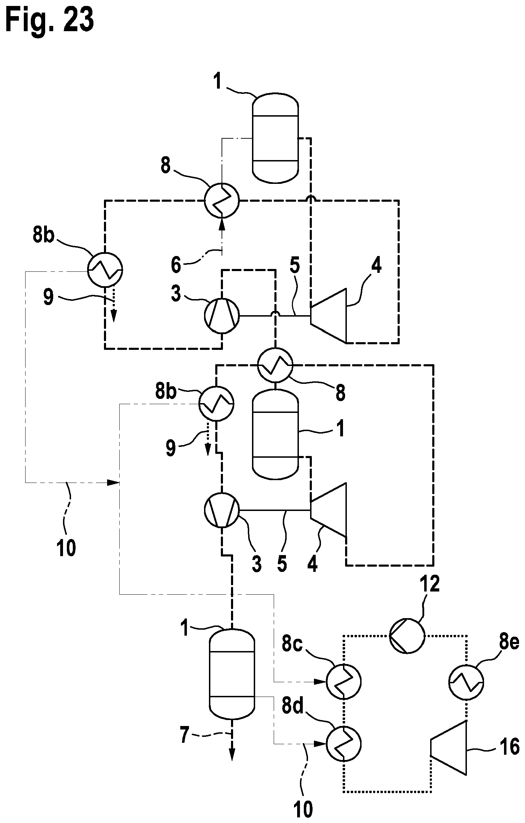

**Figure 23** shows a schematic for a system for the production of methane-enriched gas and power from hydrogen and carbon-containing starting materials according to the invention similar to the system of **Figure 16**, comprising an additional third methanation stage.

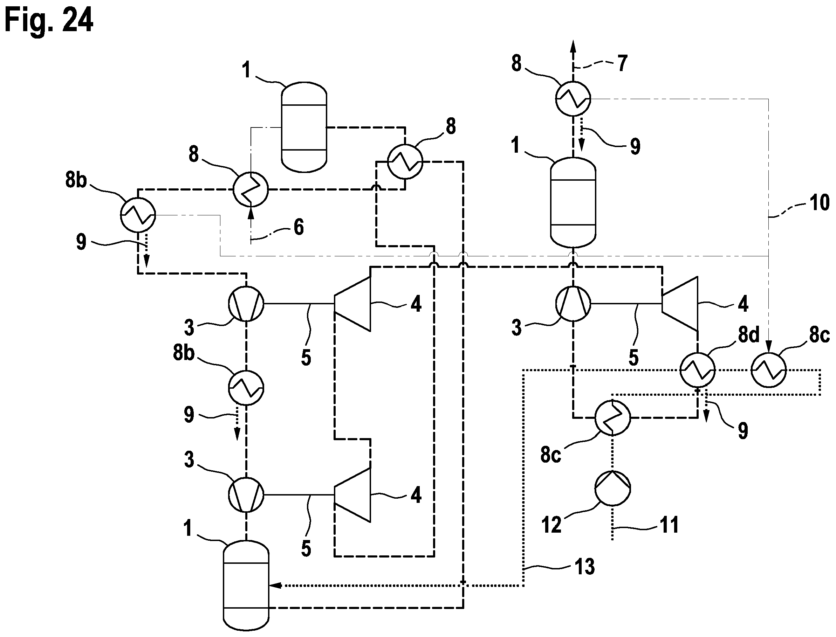

**Figure 24** shows a schematic for a complex exemplary system for the production of methane-enriched gas from hydrogen and carbon-containing starting materials according to the invention, which combines several advantages of the systems shown in previous figures.

### Description of the invention

The present invention concerns a process for the production of methane-enriched gas or methane and optionally power from hydrogen and carbon-containing starting material in a system comprising at least one single-stage or multi-stage methanation process,
comprising the following process steps:
a) providing
   one methanation reactor (1) each for the at least one single-stage or multi-stage methanation process, wherein each methanation reactor comprises an entry for the introduction of feed gas, which comprises hydrogen and carbon-containing starting materials or methane-enriched gas from a previous methanation stage, and an exit for the methane-enriched product gas;
   a continuous gas line having an entrance (6) for the introduction of hydrogen and carbon-containing starting material, and an exit (7) for the removal of the methane-enriched gas from the system; and
   at least one turbocharger (2) comprising a compressor (3) and a turbine (4) mechanically connected by a common shaft (5), wherein the methanation reactor(s) and the at least one turbocharger are connected via the continuous gas line;
   wherein the at least one compressor is connected with the continuous gas line, and is arranged in a section of the continuous gas line, which defines a path from the introduction of the starting material to the removal of the methane-enriched gas, in particular upstream, between, or downstream of the methanation reactor(s);
b) introducing the hydrogen and carbon-containing starting material into the entrance of the continuous gas line,
c) producing methane-enriched gas in the methanation reactor(s); and
d) increasing the system pressure within the continuous gas line via the at least one compressor by using the energy released during the methanation process.

The present invention is also concerned with a system for the production of methane-enriched gas or methane and optionally power from hydrogen and carbon-containing starting materials in at least one single-stage or multi-stage methanation process. The present invention is also concerned with a system for maintaining the production of methane-enriched gas or methane and power from hydrogen and carbon-containing starting materials in at least one single-stage or multi-stage methanation process.

The system is used in the process of the present invention and comprises:
one methanation reactor (1) each for the at least one single-stage or multi-stage methanation process, wherein each methanation reactor comprises an entry for the introduction of feed gas, which comprises hydrogen and carbon-containing starting materials or methane-enriched gas from a previous methanation stage, and an exit for the methane-enriched product gas;
a continuous gas line having an entrance (6) for the introduction of hydrogen and carbon-containing starting material, and an exit (7) for the removal of the methane-enriched gas from the system; and
at least one turbocharger (2) comprising a compressor (3) and a turbine (4) mechanically connected by a common shaft (5);
wherein the at least one compressor is connected with the continuous gas line, and is arranged in a section of the continuous gas line which defines a path from the introduction of the starting material to the removal of the methane-enriched gas, in particular upstream, between, or downstream of the methanation reactor(s).

Another aspect of the present invention is the use of the system of the present invention for the methanation process of the present invention.

With the process or system of the present invention, a methanation process can be maintained efficiently by using the heat generated in the methanation reactor to drive a turbocharger to increase the pressure in the system. Therefore, the present invention can also be described as being a process or system for maintaining the production of methane-enriched gas or methane and optionally power from hydrogen and carbon-containing starting materials in a system comprising at least one single-stage or multi-stage methanation process as described above.

A feed gas in the context of the present invention is the gas that enters the methanation reactor, and, prior to the first methanation stage, the feed gas entering the methanation reactor comprises hydrogen and the carbon-containing starting materials. The feed gas in subsequent methanation stages comprises the product gas of the previous methanation stage, which is the methane-enriched gas produced in the previous methanation stage. The product gas in the context of the present invention is the gas exiting a methanation reactor. The product gas is methane-enriched or methanized gas, wherein the content of methane is increases compared to the feed gas entering the same methanation reactor. Depending on the composition of the feed, gas, for example the content of methane in the feed gas, and the reaction condition in the methanation reactor, the methane-enriched product gas can have a methane concentration of more than about 97%.The carbon-containing starting materials are preferably CO₂ and/or CO.

The process and the system can be operated with the parameters used by the person skilled in the art.

The carbon-containing starting materials are preferably provided at atmospheric pressure. By using the compressor according to the invention, the pressure at the exit of the continuous gas line is higher than the pressure at the entrance of the continuous gas line. Preferably, the pressure can range from 0.1 - 100 bar, more preferably from 1.5 - 25 bar and even more preferably from 2 - 8 bar. In a generic setting, the compressor of a turbocharger increases the pressure by a factor of 2 to 8, and the turbine reduces the pressure by a factor of 1.5 to 3. The specific performance depends on the quality of the turbocharger. With every turbocharger stage, a pressure increase of a factor of about 1.5 to 3 can be expected. In the system shown in Example 1 and Figure 1, the entry pressure is 1 bar. After the compressor, the pressure is increased to 3 bar. The methanation process occurs at 3 bar. In the turbine, the pressure decreases to 1.5 bar. The product gas exits the system with a pressure of 1.5 bar.

In a two-stage system such as shown in Example 12 and Figure 12, the pressure of 3 bar after the first compressor increases to 9 bar after the second compressor (factor of 3 for each stage). The first methanation process occurs at 9 bar. In the first turbine, the pressure decreases to 4.5 bar. The second methanation process occurs at 4.5 bar at decreased temperature. In the second turbine, the pressure decreases to 2.25 bar. The product gas exits the system with a pressure of 2.25 bar.

The process and the system of the present invention are particularly efficient in maintaining an ongoing methanation process. They can however also be used when a new methanation reaction is started. To start a new methanation reaction, the process of the present invention can further comprise preheating the methanation reactor(s) prior to introducing the hydrogen and carbon-containing starting materials into the entrance of the continuous gas line. The temperature range of the starting materials at the entrance of the continuous gas line can range from ambient temperature, such as room temperature, up to 100 °C. The temperature at the entrance of the first methanation reactor is preferably at least about 130 to 150 °C, as at this temperature range the exothermic reaction in the reactor maintains itself. The methanation process starts with a reactor temperature of about 200°C, more preferably of about 250°C. The feed gas comprising the hydrogen and carbon-containing starting materials is heated electrically to about 200°C and fed into the system, which allows the process to start.

The process of the present invention can further comprise an additional step between steps b) and c) of injecting water or steam directly into at least one methanation reactor, or into the continuous gas line upstream of the entry for the introduction of feed gas of the at least one methanation reactor. This provides the advantage of increasing the mass flow in the turbine compared to the compressor, allowing the compressor to provide an increased pressurizing ratio. Furthermore, the temperature in the reactor can be limited if desired by injection of steam or water into the methanation reactor directly or prior to the entry of the methanation reactor. In these embodiments comprising a water/steam injection step, the system of the present invention comprises a water/steam injection unit for directly injecting water or steam into the at least one methanation reactor, or into the continuous gas line upstream of the entry for the introduction of feed gas. The water/steam injection unit comprises a line carrying water and/or steam. The water is pumped by a pump towards the methanation reactor or the continuous gas line at location prior to the entry of the methanation reactor. The water is converted to steam or hot water in a heat exchanger element using the heat provided by the product gas prior to the product gas exiting the system.

The process of the present invention can further comprise, additionally or alternatively, providing the compressor with external energy to facilitate the initial compression of the hydrogen and the carbon-containing starting materials prior to the start of the methanation process.

The location of the compressor in the process and system of the present invention is not particularly limited as long as the compressor is located at a position where it can pressurize gas in the continuous gas line. In one alternative, the compressor increases the pressure within the continuous gas line at a position located between the entrance of the continuous gas line and the entry of the methanation reactor, i.e. the compressor can be located at a position in the continuous gas line, which is located prior to the methanation reactor(s). This provides the advantage of having the methanation process occurring at an increased pressure level. In another alternative, the compressor increases the pressure within the continuous gas line at a position located between the exit of the methanation reactor and the exit of the continuous gas line, i.e. the compressor can also be located at a position in the continuous gas line, which is located after the methanation reactor(s). This provides the advantage that the volume flow in the compressor is reduced. In the methanation reaction, four molecules H₂ and one molecule CO₂ are reacted to two molecules water and one molecule methane, which corresponds to a reduction in volume to about 3/5. Reduction of volume flow in the compressor allows increase of the compressor performance.

In another alternative, the turbine can be located at a position upstream of the compressor. This provides the advantage of reducing the volume flow in the compressor further. The gas leaving the turbine can be cooled and water vapor can be condensed and removed prior to the gas entering the compressor. Thereby the gas volume is further reduced, particularly when comparing gas volume in the turbine and gas volume in the compressor.

In embodiments with more than one turbocharger, one compressor can be located prior to the first methanation reactor, and another compressor can be located after the last methanation reactor. One or more compressors can also be located between any two methanation reactors.

In the process or system of the present invention, the at least one turbocharger can be located upstream of the methanation reactor and heat produced in the methanation reactor can be supplied to the turbocharger at a location between the turbine and the compressor via at least one heat exchanger. Alternatively, the at least one turbocharger can be located downstream of the methanation reactor and heat produced in the methanation reactor can be supplied to the turbocharger at a location between the turbine and the compressor via at least one heat exchanger. The heat exchanger ensures that heat can be transferred to the turbocharger between its compressor and turbine components even in cases where the turbocharger is located upstream or downstream of the methanation reactor. Alternatively, the at least one turbocharger can be located between two methanation reactors, and heat produced in the methanation reactor located upstream of the turbocharger is supplied to the turbocharger prior to the turbocharger turbine inlet, preferably at a location between the turbine and the compressor, via at least one heat exchanger.

The process or system of the present invention comprise at least one turbocharger. They can also comprise a plurality of turbochargers. In certain embodiments 2, 3, or more turbochargers can be used. With every additional turbocharger stage, the pressure in the system can be increased.

The process or system of the present invention can further be controlled by intermediate cooling or intermediate heating of the continuous gas line via heat exchanger elements.

The term "heat exchanger element/heat exchanger" as used in this description can refer to an intermediate cooling element, an intermediate heating element, a recuperator, a gas cooling element, a pre-heating element, a condenser, and/or an evaporator. In a preferred embodiment, the heat exchanger can be a recuperator, wherein the recuperator transfers heat from the gas exiting the turbine to the hydrogen and the carbon-containing starting material to enter the methanation reactor.

In the process or system of the present invention, the at least one single-stage methanation process can be adiabatic or at least one stage of the multi-stage methanation process can be adiabatic. In an adiabatic process the gas temperature increases, which enhances the performance of the turbine. In a preferred embodiment, the first stage is performed in an adiabatic methanation reactor, leading to a maximum temperature in the system. The maximum temperature reached in the reactor can range from 350 to 800 °C, such as from 500 to 800 °C, and depends on the composition of the gas and the pressure. A preferred maximum temperature ranges from about 430 to about 720 °C, such as from 630 to about 720 °C. An adiabatic process is preferably used because it allows using the heat flow generated in the adiabatic process to drive the turbocharger, and particularly the compressor of the turbocharger. Further stages can also be adiabatic.

In the process or system of the present invention, the at least one single-stage methanation process can be isothermal or at least one stage of the multi-stage methanation process can be isothermal. The isothermal reaction occurs at a lower temperature range of about 200 to 300 °C and provides the advantage of the providing a better gas composition that can be used in downstream applications without further processing. A better gas composition means that more of the starting materials are reacted to methane. The product gas of an isothermal process comprises more methane than the product gas of an adiabatic process using the same starting materials. This is the case because the reaction balance at the high temperatures reached in an adiabatic process for the reaction to methane is not optimal.

In a preferred embodiment of the invention, the process or the system comprise a combination of at least one adiabatic process and at least one isothermal process to benefit from the advantages provided by both of these processes. The number of methanation stages can be chosen by the person of skill in the art depending on the requirements for the methane-enriched gas to be produced in the system. Further stages allow increasing the concentration of methane in the product gas.

In the isothermal methanation process of the invention, the temperature in the methanation reactor is controlled by a thermal power plant.

A thermal power plant in the context of the present invention is a system in which heat energy can be converted to electric power. When the thermal power plant is present in the system or process of the present invention, the system or process of the invention can be used to produce methane-enriched gas and power. For example, a steam-driven turbine can convert heat to mechanical power as an intermediate to electrical power. The steam turbine is connected to thermal power plant lines, in which a working medium is conducted. In preferred embodiments, the working medium is water and/or steam.

Alternatively, the working medium can be thermal oil or molten salt. Thermal oil or molten salt are generally used in intermediate circuits to store heat. The heat stored in the thermal oil/molten salt circuit is then transferred to a steam circuit. Therefore, the system will comprise an additional circuit compared to a system, which uses direct cooling with water/steam, making the system more complex. On the other hand, with indirect cooling via a thermal oil/molten salt circuit, the reactor does not need to be designed to work with high pressure such as up to 80 bar, as can be the case for direct cooling with a water/steam circuit. This is the case because a thermal oil circuits does not require pressure.

In the thermal power plant, the working medium, such as water, is heated, turns into steam and drives a steam turbine, which can drive an electrical generator. In several embodiments of the present invention, the heat flow generated in an isothermal methanation reactor can be used to heat the working medium in the thermal power plant system. Additionally, the working medium can be preheated in a heat exchanger element, wherein the heated methanized gas preheats the working medium prior to the methanized gas exiting the system. After the steam passes through the turbine, the steam is condensed in a condenser and recycled to where it was heated.

In the process or system of the present invention, the turbine of the turbocharger can reduce the pressure of the gas exiting a methanation reactor. Additionally or alternatively, the turbine can be located in the steam circuit of the thermal power plant and reduce the pressure of the working medium conducted in thermal power plant lines used to control the temperature of another methanation reactor.

In the process or system of the present invention, a portion of the heat produced in the methanation process can be used in the turbocharger, and another portion, which is preferably the bigger portion, can be used to produce steam or power. As it is thermodynamically impossible to use all of the heat produced in the methanation process in the turbocharger, it is advantageous to use the remaining heat for the generation of power. This makes the process and system more efficient.

The invention is further illustrated in the following examples.

### Examples

### Example 1

One example for a system and process of the present invention is shown in **Figure 1**.

**Figure** 1 shows a schematic for a system for the production of methane-enriched gas from hydrogen and carbon-containing starting materials according to the invention. The system comprises a turbocharger comprising a compressor (3) and a turbine (4) mechanically connected by a common shaft (5), and an adiabatic (uncooled) methanation reactor (1) arranged between the compressor and the turbine. The inlet pressure is 1 bar (a), the temperature 20 °C. The feed gas consists e.g. of 4 mol/s H₂ and 1 mol/s CO₂. The hydrogen and carbon-containing starting materials pass through the compressor. The pressure at the outlet of the compressor is e.g. 3 bar (a). The isentropic efficiency of the compressor and turbine is assumed to be 90%. Then, in the adiabatic methanation reactor at 3 bar (a), methanized gas with approx. 0.48 mol/s methane and an outlet temperature of 628 °C is generated. The produced methane-enriched gas exits the methanation reactor and enters the turbine. The gas is expanded in the turbine to approx. 1.5 bar (a) in order to drive the compressor. About 17.5 kW of power is transmitted via the shaft of the turbocharger. The expanded methane-enriched gas then exits the system. In order to achieve a complete conversion of the starting material gases, at least one further methanation reactor can be added after the turbine in the example shown, which e.g. could be operated isothermally at 280 °C, wherein the temperature in the methanation is controlled via a thermal power plant.

### Example 2

**Figure 2** shows a schematic for a system for the production of methane-enriched gas from hydrogen and carbon-containing starting materials according to the invention. The system comprises a turbocharger (2) comprising a compressor (3) and a turbine (4) mechanically connected by a common shaft (5), and an adiabatic methanation reactor (1) arranged between the compressor and the turbine. The system further comprises a heat exchanger (8). The heat exchanger is a recuperator, wherein the produced methane-enriched gas heats the hydrogen and carbon-containing starting materials exiting the compressor. The recuperator (50 K Pinch) increases the temperature at the entrance into the methanation reactor to about 513 °C (compared to about 131 °C in the system shown in **Figure 1**). The methanized gas leaving the reactor has a temperature of about 702 °C (compared to about 628 °C in the system shown in **Figure 1**). This leads to a decrease in the methane production (0.26 mol/s methane at the exit of the reactor. The increased temperature however allows the turbine to work with a decreased pressure difference to drive the compressor. Therefore, the methane-enriched gas can be provided with an increased pressure of 1.7 bar (compared to 1.5 bar in the system shown in **Figure 1**). The methane-enriched gas exiting the system after passing the recuperator has a temperature of about 181 °C.

### Example 3

**Figure 3** shows a schematic for a system for the production of methane-enriched gas from hydrogen and carbon-containing starting materials according to the invention. The system comprises a turbocharger comprising a compressor (3) and a turbine (4) mechanically connected by a common shaft (5), and a methanation reactor (1) arranged downstream of the compressor and the turbine. The system further comprises a heat exchanger (8). The heat exchanger is a recuperator, wherein the produced methane-enriched gas heats the hydrogen and carbon-containing starting materials exiting the compressor.

### Example 4

**Figure 4** shows a schematic for a system for the production of methane-enriched gas from hydrogen and carbon-containing starting materials according to the invention. The system comprises a turbocharger comprising a compressor (3) and a turbine (4) mechanically connected by a common shaft (5), and a methanation reactor (1) arranged upstream of the compressor and the turbine. The system further comprises two heat exchangers (8/8a). The produced methane-enriched gas passes through one heat exchanger, which is a recuperator (8) arranged between compressor and turbine, wherein the produced methane-enriched gas exiting the compressor counter-flows the produced methane-enriched gas exiting the methanation reactor. Steam produced in the reaction is condensed in a further heat exchanging element, a gas cooling element (8a), resulting in condensate (9) exiting the system prior to the methane-enriched gas entering the compressor.

### Example 5

**Figure 5** shows a schematic for a system for the production of methane-enriched gas from hydrogen and carbon-containing starting materials according to the invention. The system is similar to the system shown **Figure 4**. Heat flow (10) from the methanation reaction is used for recuperation of the produced methane-enriched gas exiting the compressor prior to entering the turbine.

### Example 6

**Figure 6** shows a schematic for a system for the production of methane-enriched gas from hydrogen and carbon-containing starting materials according to the invention. The system comprises a turbocharger with an inverted flow of the produced methane-enriched gas, which enters the turbine prior to entering the compressor of the turbocharger. Produced methane-enriched gas enters the turbine, and is then cooled in a recuperator by counterflowing hydrogen and carbon-containing starting materials. Steam produced in the reaction is condensed in a further heat exchanging element, an intermediate cooling element (8b), resulting in condensate (9) exiting the system prior to the methane-enriched gas entering the compressor. The methane-enriched gas exiting the compressor exits the system.

### Example 7

**Figure 7** shows a schematic for a system for the production of methane-enriched gas from hydrogen and carbon-containing starting materials according to the invention similar to the system of **Figure** 1. It further comprises water/steam injection unit comprising lines, in which water is conducted. Water (11) pumped by a pump (12) through a heat exchanger (8c/8d), which can be a pre-heating element (8c) or an evaporator (8d), wherein steam produced in the methanation reaction is condensed and the condensate exits the system, is heated to obtain hot water/steam (13), which is injected into the methanation reactor.

### Example 8

**Figure** 8 shows a schematic for a system for the production of methane-enriched gas from hydrogen and carbon-containing starting materials according to the invention similar to the system of **Figure 7**, but comprising two methanation reactors. The first methanation reactor is an adiabatic methanation reactor. The heated methane-enriched gas produced in the first methanation reactor is used to heat the methane-enriched gas leaving the second methanation reactor prior to the methane-enriched gas entering the turbine. The second methanation reactor is cooled by a boiling water cooler. The methanation process in the second methanation reactor is isothermal. The water/steam injection unit is similar to the water/steam injection unit shown in **Figure** 7. The pumped water is heated to hot water (14) in a pre-warming heat exchanger using the residual heat of the produced methane-enriched gas prior to exiting the system, and the hot water is heated to obtain steam (15) in a further heat exchanger using the heat flow produced in the second methanation reactor. The steam is injected into the continuous gas line at a point downstream of the second methanation reactor but upstream of the turbine.

### Example 9

**Figure** 9 shows a schematic for a system for the production of methane-enriched gas and power from hydrogen and carbon-containing starting materials according to the invention comprising two methanation reactors. The first methanation reactor is cooled by a boiling water cooler. The methanation process in the first methanation reactor is isothermal. The second methanation reactor is an adiabatic methanation reactor. The heated methane produced in the first methanation reactor is used to heat water in a thermal power plant comprising a steam circuit. In one part of the circuit, water is pumped through thermal power plant lines to a heat exchanger and heated by counterflowing the water with methane-enriched gas produced in the first methanation reactor. Condensate from the methane-enriched gas stream is exiting the system at this point of the circuit. In another part of the circuit, water is pumped through thermal power plant lines to another heat exchanger and heated by counterflowing the water with methane-enriched gas produced in the second methanation reactor. The heated water from both parts of the circuit enters a further heat exchanger, resulting in the creation of steam. Heat flow from the first methanation reactor is used for heating the water, resulting in turn in a cooling of the first methanation reactor. The steam is used to drive the turbine (4) of the turbocharger. Residual pressure is used in a steam turbine for power generation (16). Residual steam is cooled in a condenser and fed to the pump, thereby completing the circuit. The power generated by the steam turbine may be partly used to drive the pump of the steam circuit and/or for other purposes, such as export to the electricity grid. The hydrogen and carbon-containing starting materials enter the first methanation reactor. Produced methane-enriched gas exits the first methanation reaction, is cooled in a heat exchanger, resulting in condensate leaving the system. Then the methane-enriched gas enters the compressor prior to entering the second methanation reactor. Produced methane-enriched gas exits the second methanation reactor, is used to heat water flowing from the pump to the turbine of the turbocharger, and then exits the system.

### Example 10

**Figure** 10 shows a schematic for a system for the production of methane-enriched gas from hydrogen and carbon-containing starting materials according to the invention similar to the system of **Figure** 1, but comprising two turbochargers, wherein an intermediate cooling element is arranged between the compressors of the respective turbochargers.

### Example 11

**Figure 11** shows a schematic for a system for the production of methane-enriched gas from hydrogen and carbon-containing starting materials according to the invention similar to the system of **Figure 10**, but comprising a second adiabatic methanation reactor arranged between the turbines of the respective turbochargers.

### Example 12

**Figure 12** shows a schematic for a system for the production of methane-enriched gas from hydrogen and carbon-containing starting materials according to the invention similar to the system of **Figure 11**, but comprising an additional recuperator, wherein, prior to exiting the system, the produced methane-enriched gas is used to heat the hydrogen and carbon-containing starting materials prior to entering the first methanation reactor.

### Example 13

**Figure 13** shows a schematic for a system for the production of methane-enriched gas and power from hydrogen and carbon-containing starting materials according to the invention similar to the system of **Figure 12**, but comprising a third methanation reactor, wherein an isothermal methanation process occurs. The third methanation reactor is interacting with a thermal power plant comprising a steam turbine for power generation using the heat flow produced in the third methanation reactor.

### Example 14

**Figure 14** shows a schematic for a system for the production of methane-enriched gas from hydrogen and carbon-containing starting materials according to the invention comprising two turbochargers, wherein an intermediate cooling element (8b) is arranged between the compressors of the respective turbochargers. The hydrogen and carbon-containing starting materials are heated in a recuperator using the produced methane-enriched gas exiting the methanation reactor. The methanation reactor is arranged downstream from the turbochargers.

### Example 15

**Figure 15** shows a schematic for a system for the production of methane-enriched gas from hydrogen and carbon-containing starting materials according to the invention similar to the system of **Figure 14**, but comprising an additional adiabatic methanation reactor. The methane-enriched gas produced in the second methanation reactor is used to heat the hydrogen and carbon-containing starting materials at a position between the two turbines.

### Example 16

**Figure 16** shows a schematic for a system for the production of methane-enriched gas from hydrogen and carbon-containing starting materials according to the invention comprising a two-stage methanation process with recuperation and turbochargers, which are located downstream of their respective methanation reactors, wherein the methane-enriched gas produced in each methanation reactor enters the turbine prior to entering the compressor (inverted flow).

### Example 17

**Figure 17** shows a schematic for a system for the production of methane-enriched gas and power from hydrogen and carbon-containing starting materials according to the invention, which is a combination of a first stage corresponding to the system of **Figure 1**, a second stage with an inverted flow of the methane-enriched gas produced in the second methanation reactor in a downstream turbocharger, a third stage comprising a third methanation reactor with an isothermal methanation process controlled by a steam circuit, and a fourth methanation reactor as polisher to obtain a very high methane content in the methane-enriched gas exiting the system.

### Example 18

**Figure 18** shows a schematic for a system for the production of methane-enriched gas from hydrogen and carbon-containing starting materials according to the invention, comprising a turbocharger located downstream of the methanation reactor. The heat flow of the methanation reactor is used to heat the methane-enriched gas exiting the compressor prior to entering the turbine. The methane-enriched gas produced in the methanation reactor is cooled prior to entering the compressor. Condensate is exiting the system. A recuperator is used to heat the hydrogen and carbon-containing starting materials prior to entering the methanation reactor.

### Example 19

**Figure 19** shows a schematic for a system for the production of methane-enriched gas from hydrogen and carbon-containing starting materials according to the invention similar to the system of **Figure 18** comprising an additional recuperator arranged between the compressor and the heat exchanger using the heat flow of the methanation reactor.

### Example 20

**Figure 20** shows a schematic for a system for the production of methane-enriched gas and power from hydrogen and carbon-containing starting materials according to the invention similar to the system of **Figure 18** comprising an additional methanation stage. The additional methanation reactor is cooled by a thermal power plant by using the heat flow to vaporize water in the steam circuit of the thermal power plant to generate power.

### Example 21

**Figure 21** shows a schematic for a system for the production of methane-enriched gas from hydrogen and carbon-containing starting materials according to the invention similar to the system of **Figure 18**, but comprising an adiabatic methanation reactor and a downstream heat exchanger. The methane-enriched gas exiting the methanation reactor is used to heat the methane-enriched gas exiting the compressor.

### Example 22

**Figure 22** shows a schematic for a system for the production of methane-enriched gas and power from hydrogen and carbon-containing starting materials according to the invention similar to the system of **Figure 21** comprising an additional methanation stage. The additional methanation reactor is cooled by a thermal power plant by using the heat flow to vaporize water in the steam circuit of the thermal power plant to generate power.

### Example 23

**Figure 23** shows a schematic for a system for the production of methane-enriched gas and power from hydrogen and carbon-containing starting materials according to the invention similar to the system of **Figure 16**, comprising an additional third methanation stage. The additional third methanation reactor is cooled by a thermal power plant by using the heat flow to vaporize water in the steam circuit of the thermal power plant to generate power.

### Example 24

**Figure 24** shows a schematic for a complex exemplary system for the production of methane-enriched gas from hydrogen and carbon-containing starting materials according to the invention. An adiabatic methanation stage is followed by recuperative use of heat flow produced in the first methanation reactor, followed by intermediate cooling leading the condensate exiting the system, followed by two-stage compression in the compressors of two turbochargers. A second adiabatic methanation stage with injection of hot water/steam is followed by recuperative heating and an expansion in the turbines of the respective turbochargers. After another expansion step in the turbine of a third turbocharger, the methane is compressed in the compressor of the third turbocharger and enters a third methanation reactor. The heat flow of the third methanation reactor is used to produce steam for injection into the second methanation reactor. The advantage of this complex system is the significantly increased mass flow through the expansion turbines, whereby a very high outlet pressure can be achieved at the end of the system, e.g. up to 50 bar (a) at an inlet pressure of 1 bar (a). Due to the two-stage compression prior to the second methanation stage, a pressure of about ~5-20 bar (a) can be obtained. In combination with the intermediate cooling and condensate separation, a very high methane concentration (>97%) can be achieved. Another advantage is that a complex cooling circuit is not required, as the methanation stages are adiabatic. Since the first (high-temperature) stage is only operated at ambient pressure, the outlet temperature is inherently limited to approx. 600 °C.

### Reference Sign List

1: Methanation reactor
2: Turbocharger
3: Compressor
4: Turbine
5: Shaft
6: Entrance of continuous gas line for the introduction of hydrogen and carbon-containing starting materials (feed gas)
7: Exit for the removal of the methane-enriched gas produced (product gas)
8: Heat exchanger/recuperator
   8a: gas cooling element
   8b: intermediate cooling element
   8c: pre-heating element
   8d: evaporator
   8e: condenser
9: Exiting condensate
10: Heat flow
11: Water
12: Pump
13: Hot water/Steam
14: Hot water
15: Steam
16: Steam turbine for power generation

## Claims

1. Process for the production of methane-enriched gas from hydrogen and carbon-containing starting material in a system comprising at least one single-stage or multi-stage methanation process,
comprising the following process steps:
a) providing
one methanation reactor (1) each for the at least one single-stage or multi-stage methanation process, wherein each methanation reactor comprises an entry for the introduction of feed gas, which comprises hydrogen and carbon-containing starting materials or methane-enriched gas from a previous methanation stage, and an exit for the methane-enriched product gas;
a continuous gas line having an entrance (6) for the introduction of hydrogen and carbon-containing starting material, and an exit (7) for the removal of the methane-enriched gas from the system; and
at least one turbocharger (2) comprising a compressor (3) and a turbine (4) mechanically connected by a common shaft (5), wherein the methanation reactor(s) and the at least one turbocharger are connected via the continuous gas line;
wherein the at least one compressor is connected with the continuous gas line, and is arranged in a section of the continuous gas line, which defines a path from the introduction of the starting material to the removal of the methane-enriched gas, in particular upstream, between, or downstream of the methanation reactor(s);
b) introducing the hydrogen and carbon-containing starting material into the entrance of the continuous gas line,
c) producing methane-enriched gas in the methanation reactor(s); and
d) increasing the system pressure within the continuous gas line via the at least one compressor by using the energy released during the methanation process.

2. Process of claim 1, further comprising an additional step between steps b) and c) of injecting water or steam directly into at least one methanation reactor, or into the continuous gas line upstream of the entry for the introduction of feed gas of the at least one methanation reactor and/or turbocharger turbine.

3. Process of claim 1 or 2, wherein in step a) a thermal power plant with a working medium conducted in thermal power plant lines is provided, wherein the thermal power plant is coupled to at least one of the methanation reactors or a gas stream derived therefrom via at least one heat exchanger (8), and wherein the thermal power plant produces electrical power, wherein optionally the thermal power plant is a steam turbine (16) with steam (15) conducted in lines of a steam turbine cycle.

4. Process of any one of the preceding claims, wherein the turbine of the at least one turbocharger is connected either to the continuous gas line or, when present, to one of the thermal power plant lines; and/or further comprising step e) of removing the methane-enriched gas at the exit of the continuous gas line from the system; and/or wherein the carbon-containing starting material is CO₂ and/or CO; and/or wherein the hydrogen and the carbon-containing starting material is provided at atmospheric pressure; and/or wherein the pressure at the exit of the continuous gas line is higher than the pressure at the entrance of the continuous gas line.

5. Process of any one of the preceding claims, further comprising preheating the methanation reactor(s) prior to step b) of claim 1; and/or further comprising providing the compressor with external energy to facilitate the initial compression of the hydrogen and the carbon-containing starting material prior to the methanation process.

6. Process of any one of the preceding claims, wherein the one or more compressors increase the pressure within the continuous gas line at a position located between the entrance of the continuous gas line and the entry of the first methanation reactor; and/or wherein the one or more compressors increase the pressure within the continuous gas line at a position located between the exit of the methanation reactor and the exit of the continuous gas line; and/or wherein the turbine is located at a position upstream of the one or more compressors.

7. Process of any one of the preceding claims, wherein a plurality of turbochargers is used, wherein preferably 2, 3, or more turbochargers are used; or wherein intermediate cooling or intermediate heating of the continuous gas line is performed via heat exchanger elements (8); and/or wherein the at least one single-stage methanation process is adiabatic or at least one stage of the multi-stage methanation process is adiabatic.

8. Process of any one of claims 2 to 7, wherein the at least one single-stage methanation process is isothermal or at least one stage of the multi-stage methanation process is isothermal, wherein in the isothermal process, the temperature in the methanation reactor is controlled by the thermal power plant.

9. Process of any one of the preceding claims, wherein the turbine reduces the pressure of the gas exiting the methanation reactor; or wherein the turbine reduces the pressure of the working medium conducted in thermal power plant lines used to control the temperature of a methanation reactor, when present; and/or wherein the heat exchanger (8), when present, is a recuperator, wherein the recuperator transfers heat from the gas exiting the turbine to the hydrogen and the carbon-containing starting material prior to the hydrogen and the carbon-containing starting material entering the methanation reactor.

10. Process of any one of the preceding claims, wherein the at least one turbocharger is located upstream of the methanation reactor and heat produced in the methanation reactor is supplied to the turbocharger at a location between the turbine and the compressor via at least one heat exchanger; or wherein the at least one turbocharger is located downstream of the methanation reactor and heat produced in the methanation reactor is supplied to the turbocharger at a location between the turbine and the compressor via at least one heat exchanger; or wherein the at least one turbocharger is located between two methanation reactors, and heat produced in the methanation reactor located upstream of the turbocharger is supplied to the turbocharger prior to the turbocharger turbine inlet, preferably at a location between the turbine and the compressor, via at least one heat exchanger.

11. Process of any one of the preceding claims, wherein a portion of the heat produced in the methanation process is used in the turbocharger, and another portion, which is preferably the bigger portion, is used to produce steam or power.

12. System for the production of methane-enriched gas from hydrogen and carbon-containing starting material in at least one single-stage or multi-stage methanation process, wherein the system comprises:
one methanation reactor (1) each for the at least one single-stage or multi-stage methanation process, wherein each methanation reactor comprises an entry for the introduction of feed gas, which comprises hydrogen and carbon-containing starting materials or methane-enriched gas from a previous methanation stage, and an exit for the methane-enriched product gas;
a continuous gas line having an entrance (6) for the introduction of hydrogen and carbon-containing starting material, and an exit (7) for the removal of the methane-enriched gas from the system; and
at least one turbocharger (2) comprising a compressor (3) and a turbine (4) mechanically connected by a common shaft (5);
wherein the at least one compressor is connected with the continuous gas line, and is arranged in a section of the continuous gas line which defines a path from the introduction of the starting material to the removal of the methane-enriched gas, in particular upstream, between, or downstream of the methanation reactor(s).

13. System of claim 12, further comprising a water/steam injection unit for directly injecting water or steam into the at least one methanation reactor, or into the continuous gas line upstream of the entry for the introduction of feed gas.

14. System of claim 12 or 13, further comprising a thermal power plant with a working medium conducted in thermal power plant lines, in particular a steam turbine (16) with lines of a steam turbine cycle, wherein the methanation reactor(s) and the at least one turbocharger are connected via the continuous gas line, wherein the thermal power plant is coupled to at least one of the methanation reactors or a gas stream derived therefrom via at least one heat exchanger (8), and wherein the thermal power plant is configured to produce electrical power.

15. System of claim 12, 13, or 14, wherein the at least one turbine is connected either to the continuous gas line or, when present, to one of the thermal power plant lines.

16. Use of the system of any of claims 12 to 15 for the process of any one of claims 1 to 11.

## Patentansprüche

1. Verfahren zur Herstellung von methanangereichertem Gas aus Wasserstoff und kohlenstoffhaltigem Ausgangsmaterial in einem System umfassend mindestens ein einzelstufiges oder mehrstufiges Methanisierungsverfahren, umfassend die folgenden Verfahrensschritte:
a) Bereitstellen
eines Methanisierungsreaktors (1) jeweils für das mindestens eine einzelstufige oder mehrstufige Methanisierungsverfahren, wobei jeder Methanisierungsreaktor einen Eingang für die Zufuhr von Zufuhrgas, das Wasserstoff und kohlenstoffhaltige Ausgangsmaterials oder methanangereichertes Gas von einer vorhergehenden Methanisierungsstufe umfasst, und einen Ausgang für das methanangereicherte Produktgas umfasst;
einer kontinuierlichen Gasleitung mit einem Eingang (6) für die Zufuhr von Wasserstoff und kohlenstoffhaltigem Ausgangsmaterial und einem Ausgang (7) für die Abfuhr des methanangereicherten Gases von dem System; und
mindestens eines Turboladers (2), der einen Kompressor (3) und eine Turbine (4) umfasst, die durch eine gemeinsame Welle (5) mechanisch verbunden sind, wobei der/die Methanisierungsreaktor(en) und der mindestens eine Turbolader durch die kontinuierliche Gasleitung verbunden sind;
wobei der mindestens eine Kompressor mit der kontinuierlichen Gasleitung verbunden ist und in einem Abschnitt der kontinuierlichen Gasleitung angeordnet ist, der einen Weg von der Zufuhr des Ausgangsmaterials zur Abfuhr des methanangereicherten Gases definiert, insbesondere stromaufwärts, zwischen oder stromabwärts des Methanisierungsreaktors/der Methanisierungsreaktoren;
b) Einführen des Wasserstoffs und des kohlenstoffhaltigen Ausgangsmaterials in den Eingang der kontinuierlichen Gasleitung,
c) Erzeugen von methanangereichertem Gas in dem/den Methanisierungsreaktor(en); und
d) Erhöhen des Systemdrucks innerhalb der kontinuierlichen Gasleitung durch den mindestens einen Kompressor unter Nutzung der während des Methanisierungsverfahrenes freigesetzten Energie.

2. Verfahren nach Anspruch 1, das ferner einen zusätzlichen Schritt zwischen den Schritten b) und c) umfasst, bei dem Wasser oder Dampf direkt in mindestens einen Methanisierungsreaktor oder in die kontinuierliche Gasleitung stromaufwärts des Eingangs zur Zufuhr des Zufuhrgases des mindestens einen Methanisierungsreaktors und/oder der Turboladerturbine eingespritzt wird.

3. Verfahren nach Anspruch 1 oder 2, wobei in Schritt a) ein Wärmekraftwerk mit einem in Wärmekraftwerksleitungen geführten Arbeitsmedium bereitgestellt wird, wobei das Wärmekraftwerk durch mindestens einen Wärmetauscher (8) mit mindestens einem der Methanisierungsreaktoren oder einem daraus abgeleiteten Gasstrom gekoppelt ist, und wobei das Wärmekraftwerk elektrische Energie erzeugt, wobei das Wärmekraftwerk optional eine Dampfturbine (16) mit in Leitungen eines Dampfturbinenkreislaufs geführtem Dampf (15) ist.

4. Verfahren nach einem der vorhergehenden Ansprüche, wobei die Turbine des mindestens einen Turboladers entweder an die kontinuierliche Gasleitung oder, sofern vorhanden, an eine der Wärmekraftwerksleitungen verbunden ist; und/oder ferner umfassend Schritt e) zum Entfernen des methanangereicherten Gases am Ausgang der kontinuierlichen Gasleitung von dem System; und/oder wobei das kohlenstoffhaltige Ausgangsmaterial CO₂ und/oder CO ist; und/oder wobei der Wasserstoff und das kohlenstoffhaltige Ausgangsmaterial bei atmosphärischem Druck bereitgestellt werden; und/oder wobei der Druck am Ausgang der kontinuierlichen Gasleitung höher ist als der Druck am Eingang der kontinuierlichen Gasleitung.

5. Verfahren nach einem der vorhergehenden Ansprüche, ferner umfassend ein Vorwärmen des/der Methanisierungsreaktors/-reaktoren vor Schritt b) von Anspruch 1; und/oder ferner umfassend ein Bereitstellen des Kompressors mit externer Energie, um die anfängliche Kompression des Wasserstoffs und des kohlenstoffhaltigen Ausgangsmaterials vor dem Methanisierungsverfahren zu ermöglichen.

6. Verfahren nach einem der vorhergehenden Ansprüche, wobei der eine oder die mehreren Kompressoren den Druck innerhalb der kontinuierlichen Gasleitung an einer Position erhöhen, die sich zwischen dem Eingang der kontinuierlichen Gasleitung und dem Eingang des ersten Methanisierungsreaktors befindet; und/oder wobei der eine oder die mehreren Kompressoren den Druck innerhalb der kontinuierlichen Gasleitung an einer Position erhöhen, die sich zwischen dem Ausgang des Methanisierungsreaktors und dem Ausgang der kontinuierlichen Gasleitung befindet; und/oder wobei sich die Turbine an einer Position stromaufwärts des einen oder der mehreren Kompressoren befindet.

7. Verfahren nach einem der vorhergehenden Ansprüche, wobei eine Mehrzahl von Turboladern verwendet werden, wobei vorzugsweise 2, 3 oder mehr Turboladern verwendet werden; oder wobei eine Zwischenkühlung oder Zwischenerwärmung der kontinuierlichen Gasleitung durch Wärmetauscherelemente (8) erfolgt; und/oder wobei das mindestens eine einzelstufige Methanisierungsverfahren oder mindestens eine Stufe des mehrstufigen Methanisierungsverfahrenes adiabat ist.

8. Verfahren nach einem der Ansprüche 2 bis 7, wobei das mindestens eine einzelstufige Methanisierungsverfahren isotherm ist oder die mindestens eine Stufe des mehrstufigen Methanisierungsverfahrenes isotherm ist, wobei im isothermen Verfahren die Temperatur im Methanisierungsreaktor durch das Wärmekraftwerk gesteuert wird.

9. Verfahren nach einem der vorhergehenden Ansprüche, wobei die Turbine den Druck des von dem Methanisierungsreaktor austretenden Gases verringert; oder wobei die Turbine den Druck des Arbeitsmediums verringert, das in Wärmekraftwerksleitungen geführt wird, die zur Steuerung der Temperatur eines Methanisierungsreaktors verwendet werden, sofern vorhanden; und/oder wobei der Wärmetauscher (8), sofern vorhanden, ein Rekuperator ist, wobei der Rekuperator Wärme von dem von der Turbine austretenden Gas auf den Wasserstoff und das kohlenstoffhaltige Ausgangsmaterial überträgt, bevor der Wasserstoff und das kohlenstoffhaltige Ausgangsmaterial in den Methanisierungsreaktor eintreten.

10. Verfahren nach einem der vorhergehenden Ansprüche, wobei sich der mindestens eine Turbolader stromaufwärts des Methanisierungsreaktors befindet und im Methanisierungsreaktor erzeugte Wärme dem Turbolader an einer Stelle zwischen der Turbine und dem Kompressor durch mindestens einen Wärmetauscher zugeführt wird; oder wobei sich der mindestens eine Turbolader stromabwärts des Methanisierungsreaktors befindet und im Methanisierungsreaktor erzeugte Wärme dem Turbolader an einer Stelle zwischen der Turbine und dem Kompressor durch mindestens einen Wärmetauscher zugeführt wird; oder wobei sich der mindestens eine Turbolader zwischen zwei Methanisierungsreaktoren befindet und im stromaufwärts des Turboladers befindlichen Methanisierungsreaktor erzeugte Wärme dem Turbolader vor dem Turbolader-Turbineneinlass, vorzugsweise an einer Stelle zwischen der Turbine und dem Kompressor, durch mindestens einen Wärmetauscher zugeführt wird.

11. Verfahren nach einem der vorhergehenden Ansprüche, wobei ein Teil der im Methanisierungsverfahren erzeugten Wärme im Turbolader verwendet wird und ein anderer Teil, vorzugsweise der größere Teil, zur Erzeugung von Dampf oder Strom verwendet wird.

12. System zur Herstellung von methanangereichertem Gas aus Wasserstoff und kohlenstoffhaltigem Ausgangsmaterial in mindestens einem einzelstufigen- oder mehrstufigen Methanisierungsverfahren, wobei das System umfasst:
einen Methanisierungsreaktor (1)jeweils für das mindestens eine einzelstufige- oder mehrstufige Methanisierungsverfahren, wobei jeder Methanisierungsreaktor einen Eingang für die Zufuhr von Zufuhrgas, das Wasserstoff und kohlenstoffhaltige Ausgangsmateriale oder methanangereichertes Gas von einer vorhergehenden Methanisierungsstufe umfasst, und einen Ausgang für das methanangereicherte Produktgas umfasst;
eine kontinuierliche Gasleitung, die einen Eingang (6) für die Zufuhr von Wasserstoff und kohlenstoffhaltigem Ausgangsmaterial und einen Ausgang (7) für die Abfuhr des methanangereicherten Gases von dem System umfasst; und
mindestens einen Turbolader (2), der einen Kompressor (3) und eine Turbine (4) umfasst, die mechanisch durch eine gemeinsame Welle (5) verbunden sind;
wobei der mindestens eine Kompressor mit der kontinuierlichen Gasleitung verbunden ist und in einem Abschnitt der kontinuierlichen Gasleitung angeordnet ist, der einen Weg von der Zufuhr des Ausgangsmaterials zur Abfuhr des methanangereicherten Gases definiert, insbesondere stromaufwärts, zwischen oder stromabwärts des Methanisierungsreaktors/der Methanisierungsreaktoren.

13. System nach Anspruch 12, ferner umfassend eine Wasser-/Dampfeinspritzeinheit zum direkten Einspritzen von Wasser oder Dampf in den mindestens einen Methanisierungsreaktor oder in die kontinuierliche Gasleitung stromaufwärts des Eingangs zur Zufuhr des Zufuhrgases.

14. System nach Anspruch 12 oder 13, ferner umfassend ein Wärmekraftwerk mit einem in Wärmekraftwerksleitungen geführten Arbeitsmedium, insbesondere eine Dampfturbine (16) mit Leitungen eines Dampfturbinenkreislaufs, wobei der Methanisierungsreaktor oder die Methanisierungsreaktoren und der mindestens eine Turbolader durch die kontinuierliche Gasleitung verbunden sind, wobei das Wärmekraftwerk durch mindestens einen Wärmetauscher (8) mit mindestens einem der Methanisierungsreaktoren oder einem daraus abgeleiteten Gasstrom gekoppelt ist, und wobei das Wärmekraftwerk zur Erzeugung elektrischer Energie konfiguriert ist.

15. System nach Anspruch 12, 13 oder 14, wobei die mindestens eine Turbine entweder an die kontinuierliche Gasleitung oder, sofern vorhanden, an eine der Wärmekraftwerksleitungen verbunden ist.

16. Verwendung des Systems nach einem der Ansprüche 12 bis 15 für das Verfahren nach einem der Ansprüche 1 bis 11.

## Revendications

1. Procédé de production de gaz enrichi en méthane à partir d'un matériau de départ contenant de l'hydrogène et du carbone dans un système comprenant au moins un procédé de méthanation à un seul étage ou à plusieurs étages,
comprenant les étapes de procédé suivantes :
a) la fourniture
d'un réacteur de méthanation (1) chacun pour l'au moins un procédé de méthanation à un seul étage ou à plusieurs étages, dans lequel chaque réacteur de méthanation comprend une entrée pour l'introduction du gaz d'alimentation, qui comprend des matériaux de départ contenant de l'hydrogène et du carbone ou du gaz enrichi en méthane provenant d'un étage de méthanation précédent, et une sortie pour le gaz de produit enrichi en méthane ;
d'une conduite de gaz continue ayant une admission (6) pour l'introduction du matériau de départ contenant de l'hydrogène et du carbone, et une sortie (7) pour l'élimination du gaz enrichi en méthane du système ; et
d'au moins un turbocompresseur (2) comprenant un compresseur (3) et une turbine (4) reliés mécaniquement par un arbre commun (5), dans lequel le(s) réacteur(s) de méthanation et l'au moins un turbocompresseur sont reliés par l'intermédiaire de la conduite de gaz continue ;
dans lequel l'au moins un compresseur est relié à la conduite de gaz continue et est agencé dans une section de la conduite de gaz continue qui définit un chemin entre l'introduction du matériau de départ et l'élimination du gaz enrichi en méthane, en particulier en amont, entre ou en aval du ou des réacteur(s) de méthanation ;
b) l'introduction du matériau de départ contenant de l'hydrogène et du carbone dans l'admission de la conduite de gaz continue,
c) la production du gaz enrichi en méthane dans le(s) réacteur(s) de méthanation ; et
d) l'augmentation de la pression du système dans la conduite de gaz continue par l'intermédiaire de l'au moins un compresseur en utilisant l'énergie libérée au cours du procédé de méthanation.

2. Procédé de la revendication 1, comprenant en outre une étape supplémentaire entre les étapes b) et c) d'injection de l'eau ou de la vapeur directement dans au moins un réacteur de méthanation, ou dans la conduite de gaz continue en amont de l'entrée pour l'introduction du gaz d'alimentation de l'au moins un réacteur de méthanation et/ou une turbine de turbocompresseur.

3. Procédé de la revendication 1 ou 2, dans lequel dans l'étape a) on prévoit une centrale thermique avec un milieu de travail acheminé dans les conduites de centrale thermique, dans lequel la centrale thermique est couplée à au moins un des réacteurs de méthanation ou à un flux de gaz dérivé de celui-ci par l'intermédiaire de l'au moins un échangeur de chaleur (8), et dans lequel la centrale thermique produit de de la puissance électrique, dans lequel facultativement la centrale thermique est une turbine à vapeur (16) avec de la vapeur (15) acheminée dans les conduites d'un cycle de turbine à vapeur.

4. Procédé de l'une quelconque des revendications précédentes, dans lequel la turbine de l'au moins un turbocompresseur est reliée soit à la conduite de gaz continue, soit, le cas échéant, à l'une des conduites de centrale thermique ; et/ou comprenant en outre l'étape e) d'élimination, du système, du gaz enrichi en méthane au niveau de la sortie de la conduite de gaz continue ; et/ou dans lequel le matériau de départ contenant du carbone est du CO₂ et/ou du CO ; et/ou dans lequel le matériau de départ contenant de l'hydrogène et du carbone est fourni à la pression atmosphérique ; et/ou dans lequel la pression au niveau de la sortie de la conduite de gaz continue est supérieure à la pression au niveau de l'admission de la conduite de gaz continue.

5. Procédé de l'une quelconque des revendications précédentes, comprenant en outre le préchauffage du ou des réacteur(s) de méthanation avant l'étape b) de la revendication 1 ; et/ou comprenant en outre la fourniture d'énergie externe au compresseur pour faciliter la compression initiale du matériau de départ contenant de l'hydrogène et du carbone avant le procédé de méthanation.

6. Procédé de l'une quelconque des revendications précédentes, dans lequel le ou les plusieurs compresseurs augmentent la pression à l'intérieur de la conduite de gaz continue à une position située entre l'admission de la conduite de gaz continue et l'entrée du premier réacteur de méthanation ; et/ou dans lequel le ou les plusieurs compresseurs augmentent la pression à l'intérieur de la conduite de gaz continue à une position située entre la sortie du réacteur de méthanation et la sortie de la conduite de gaz continue ; et/ou dans lequel la turbine est située à une position en amont du ou des plusieurs compresseurs.

7. Procédé de l'une quelconque des revendications précédentes, dans lequel une pluralité de turbocompresseurs sont utilisés, dans lequel de préférence 2, 3 turbocompresseurs ou plus sont utilisés ; ou dans lequel un refroidissement intermédiaire ou un chauffage intermédiaire de la conduite de gaz continue est effectué par l'intermédiaire d'éléments d'échangeur de chaleur (8) ; et/ou dans lequel l'au moins un procédé de méthanation à un seul étage est adiabatique ou au moins un étage du procédé de méthanation à plusieurs étages est adiabatique.

8. Procédé de l'une quelconque des revendications 2 à 7, dans lequel l'au moins un procédé de méthanation à un seul étage est isotherme ou au moins un étage du procédé de méthanation à plusieurs étages est isotherme, dans lequel dans le procédé isotherme, la température dans le réacteur de méthanation est commandée par la centrale thermique.

9. Procédé de l'une quelconque des revendications précédentes, dans lequel la turbine réduit la pression du gaz sortant du réacteur de méthanation ; ou dans lequel la turbine réduit la pression du milieu de travail acheminé dans les conduites de centrale thermique utilisées pour commander la température d'un réacteur de méthanation, le cas échéant ; et/ou dans lequel l'échangeur de chaleur (8), le cas échéant, est un récupérateur, dans lequel le récupérateur transfère la chaleur du gaz sortant de la turbine au matériau de départ contenant de l'hydrogène et du carbone avant que le matériau de départ contenant de l'hydrogène et du carbone n'entre dans le réacteur de méthanation.

10. Procédé de l'une quelconque des revendications précédentes, dans lequel l'au moins un turbocompresseur est situé en amont du réacteur de méthanation et la chaleur produite dans le réacteur de méthanation est fournie au turbocompresseur à un emplacement situé entre la turbine et le compresseur par l'intermédiaire d'au moins un échangeur de chaleur ; ou dans lequel l'au moins un turbocompresseur est situé en aval du réacteur de méthanation et la chaleur produite dans le réacteur de méthanation est fournie au turbocompresseur à un emplacement situé entre la turbine et le compresseur par l'intermédiaire d'au moins un échangeur de chaleur ; ou dans lequel l'au moins un turbocompresseur est situé entre deux réacteurs de méthanation, et la chaleur produite dans le réacteur de méthanation situé en amont du turbocompresseur est fournie au turbocompresseur avant l'entrée de la turbine de turbocompresseur, de préférence à un emplacement situé entre la turbine et le compresseur, par l'intermédiaire d'au moins un échangeur de chaleur.

11. Procédé de l'une quelconque des revendications précédentes, dans lequel une partie de la chaleur produite dans le procédé de méthanation est utilisée dans le turbocompresseur, et une autre partie, qui est de préférence la partie la plus importante, est utilisée pour produire de la vapeur ou de la puissance.

12. Système de production de gaz enrichi en méthane à partir d'un matériau de départ contenant de l'hydrogène et du carbone et dans au moins un procédé de méthanation à un seul étage ou à plusieurs étages, dans lequel le système comprend :
un réacteur de méthanation (1) chacun pour l'au moins un procédé de méthanation à un seul étage ou à plusieurs étages, dans lequel chaque réacteur de méthanation comprend une entrée pour l'introduction du gaz d'alimentation, qui comprend des matériaux de départ contenant de l'hydrogène et du carbone ou du gaz enrichi en méthane provenant d'un étage de méthanation précédent, et une sortie pour le gaz de produit enrichi en méthane ;
une conduite de gaz continue ayant une admission (6) pour l'introduction du matériau de départ contenant l'hydrogène et du carbone, et une sortie (7) pour l'élimination, du système, du gaz enrichi en méthane ; et
au moins un turbocompresseur (2) comprenant un compresseur (3) et une turbine (4) reliés mécaniquement par un arbre commun (5) ;
dans lequel l'au moins compresseur est relié à la conduite de gaz continue et est agencé dans une section de la conduite de gaz continue qui définit un chemin entre l'introduction du matériau de départ et l'élimination du gaz enrichi en méthane, en particulier en amont, entre ou en aval du ou des réacteur(s) de méthanation.

13. Système de la revendication 12, comprenant en outre une unité d'injection d'eau/de vapeur pour injecter directement de l'eau ou de la vapeur dans l'au moins un réacteur de méthanation, ou dans la conduite de gaz continue en amont de l'entrée pour l'introduction du gaz d'alimentation.

14. Système de la revendication 12 ou 13, comprenant en outre une centrale thermique avec un milieu de travail acheminé dans des conduites de centrale thermique, en particulier une turbine à vapeur (16) avec des conduites d'un cycle de turbine à vapeur, dans lequel le(s) réacteur(s) de méthanation et l'au moins un turbocompresseur sont reliés par l'intermédiaire de la conduite de gaz continue, dans lequel la centrale thermique est couplée à au moins un des réacteurs de méthanation ou à un flux de gaz dérivé de celui-ci par l'intermédiaire d'au moins un échangeur de chaleur (8), et dans lequel la centrale thermique est configurée pour produire de la puissance électrique.

15. Système de la revendication 12, 13 ou 14, dans lequel l'au moins une turbine est reliée soit à la conduite de gaz continue, soit, le cas échéant, à l'une des conduites de centrale thermique.

16. Utilisation du système de l'une des revendications 12 à 15 pour le procédé de l'une quelconque des revendications 1 à 11.
